# FASCICULE DE BREVET EUROPEEN

(11) **EP 2 456 724 B1**
(45) Date de publication et mention de la délivrance du brevet: **07.09.2016**
(21) Numéro de dépôt: 10740382.6
(22) Date de dépôt: 19.07.2010
(51) Int. Cl.: C02F 1/04, C02F 3/00, C02F 3/28, C12P 5/02, C05F 17/00, C02F 103/32, C02F 101/10

(54) **Procédé de méthanisation comprenant une séparation des métabolites inhibant ladite méthanisation**
Methanisierungsverfahren mit einer Abtrennung von Metaboliten, die hemmend auf die Methanisierung wirken
Methanisation process comprising a separation step for metabolites inhibiting said methanisation

(30) Priorité: 21.07.2009 FR 0903582
(43) Date de publication de la demande: 30.05.2012
(73) Titulaire: Ondeo Industrial Solutions, 92040 Paris La Defense (FR)
(72) Inventeur: DELPORTE, Claude, F-92500 Rueil-Malmaison (FR); MAILLARD, Sarah, F-78100 Saint-Germain-en-Laye (FR)
(74) Mandataire: Cabinet Armengaud Aîné
(86) Numéro de dépôt international: PCT/IB2010/053277
(87) Numéro de publication internationale: WO 2011/010275

(56) Documents cités:
- EP-A1- 0 245 910
- WO-A1-02/100784
- WO-A1-2008/094282
- WO-A1-2009/102916
- WO-A2-2009/076947
- FR-A1- 2 924 441
- US-A- 4 659 471
- US-A- 4 663 043
- US-A- 5 670 047
- US-A1- 2004 182 779
- Dores G. Cirne et al: "Control of sulphide during anaerobic treatment of S-containing wastewaters by adding limited amounts of oxygen or nitrate", Rev Environ Sci Biotechnol, 1 février 2008 (2008-02-01), pages 93-105, XP002572867, DOI: 10.1007/s11157-008-9128-9 Extrait de l'Internet: URL:http://www.springerlink.com/content/2l 3706587167346j/fulltext.pdf [extrait le 2010-03-11]
- F. Fernandez-Polanco et al: "Simultaneous organic nitrogen and sulfate removal in an anaerobic GAC fluidized bed reactor", Water Science and Technology, vol. 44, no. 4 2001, pages 15-22, XP002572868, Extrait de l'Internet: URL:http://lequia.udg.es/lequianet/WatSciT ech/04404/0015/044040015.pdf [extrait le 2010-03-11]
- F. Fernandez-Polanco: "Combining the biological nitrogen and sulfur cycles in anaerobic conditions", Water Science and Technology, vol. 44, no. 8 2001, pages 77-84, XP002572869, Extrait de l'Internet: URL:http://lequia.udg.es/lequianet/WatSciT ech/04408/0077/044080077.pdf [extrait le 2010-03-11]

## Description

L'invention est relative à un procédé de méthanisation, à partir d'effluents industriels ou urbains, liquides ou solides, en particulier vinasses de mélasses issues de la production d'alcools ou de levures, contenant des éléments ou des composés tels que potassium et sulfates inhibant la méthanisation.

### 1. ETAT DE LA TECHNIQUE

### 1.1 Rappel des processus élémentaires du génie biologique du traitement d'eau

### 1.1.1 Biochimie et microbiologique de la méthanogénèse

La fermentation anaérobie est réalisée par des populations bactériennes complexes qui, dans des conditions d'environnement bien précises (potentiel redox autour de -250 mV, pH voisin de la neutralité), forment des associations stables. Elle est strictement anaérobie et conduit à la formation de méthane.

En partant d'un cas simple comme le glucose, le bilan général de la digestion anaérobie peut s'écrire comme suit :

C₆H₁₂O₆+0,2 NH₃ → 0,2 C₅H₇NO₂ + 2,5 CH₄ + 2,5 CO₂ + 0,6 H₂O

La méthanisation de la matière organique a lieu dans les écosystèmes "froids" (10-15°C), mésophiles (30-40°C) et même thermophiles (>45°C).

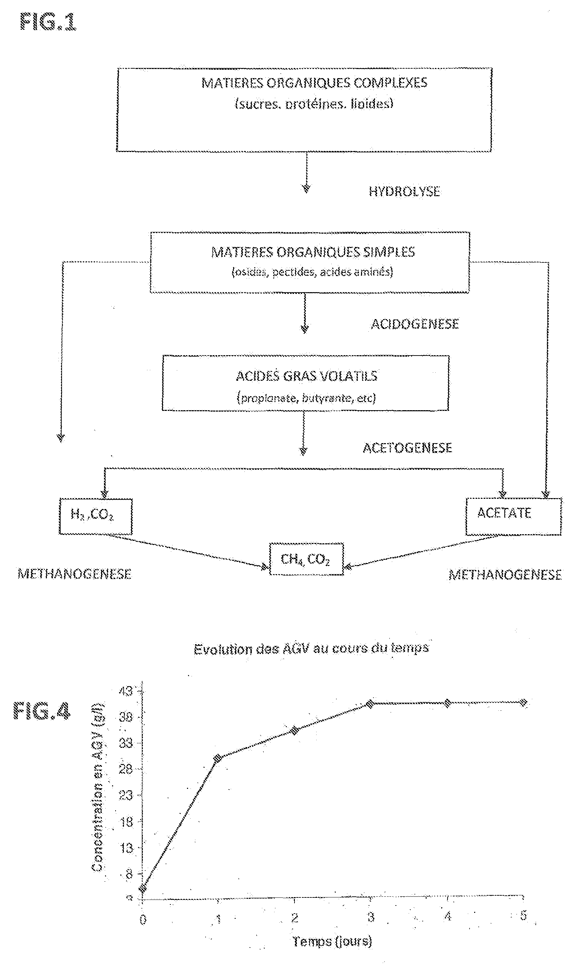

Les différentes voies de dégradation de la matière organique complexe en anaérobiose, peuvent être schématisées de la manière illustrée sur Fig.1 des dessins annexés. Les matières organiques complexes (sucres, protéines, lipides) soumise à une hydrolyse donnent des matières organiques simples (osides, pectides, acides aminés) qui peuvent donner d'une part directement de l'hydrogène H₂ et du dioxyde de carbone CO₂ et d'autre part par acidogénèse des acides gras volatils (propionate, butyrate, valérate etc.). Les acides gras volatils, par acétogénèse donnent de l'hydrogène H₂ et du dioxyde de carbone CO₂, ainsi que des acétates.

L'étape de méthanogénèse donne du méthane CH₄ et du dioxyde de carbone CO₂.

### Etape d'hydrolyse et d'acidogénèse :

Elle est réalisée par des micro-organismes d'espèces extrêmement diverses : mésophiles ou thermophiles, anaérobies strictes ou facultatives.

Cette première étape aboutit à un mélange d'acides gras volatils (AGV) : acétique, lactique, propionique, butyrique, etc..., de composés neutres (éthanol), de produits gazeux (CO₂ et H₂) et éventuellement d'ammonium et d'orthophosphate .

Ces micro-organismes acidogènes ont souvent des temps de génération plus courts que ceux réalisant les étapes suivantes de la méthanogénèse.

### La dégradation des oses :

Elle s'effectue selon la voie d'Embden et MeyerHoff conduisant à l'acide pyruvique.

Les divers types de fermentations possibles du pyruvate (alcoolique, homolactique, butyrique, propionique...) peuvent théoriquement conduire à la formation de gaz carbonique, d'hydrogène, d'éthanol, et des acides formique, acétique, propionique, butyrique et lactique.

Dans les digesteurs, le plus souvent, l'acide acétique est nettement prépondérant et n'est accompagné que des acides propionique et butyrique en quantités moindres. L'apparition d'acides gras à chaînes plus longues traduit un déséquilibre dans le fonctionnement du digesteur (Couplet et al., 1978).

### Les composés azotés :

Les acides aminés issus des protéines peuvent donner par leurs mécanismes de dégradation habituels (transamination, désamination, décarboxylation), des acides gras volatils ainsi que du gaz carbonique et de l'ammonium. Comme les glucides, les acides aminés sont susceptibles de conduire au pyruvate et donc aux mêmes produits que les oses dégradés par la glycolyse.

### Les lipides

Les mécanismes de formation d'acides gras à chaîne courte à partir d'acides gras à chaîne plus longue, font intervenir le processus de la bêta oxydation.

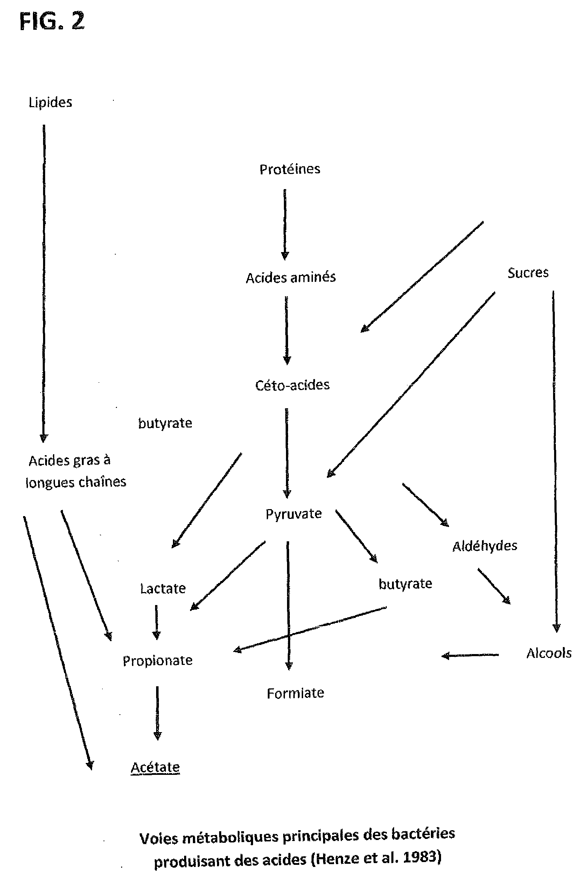

Les grands traits des voies métaboliques des bactéries produisant des acides sont regroupés sur Fig.2 des dessins annexés.

Les bactéries hydrolytiques et acidogènes sont anaérobies strictes ou facultatives et ont un pH optimum de croissance proche de 5,5 et 7 (Henze et al. 1983).

### 2. LIMITE DU PROCEDE

Après l'étape d'hydrolyse/acidogénèse, les effluents sont habituellement dirigés vers l'étape de méthanisation après avoir subi une étape de séparation et de recyclage de la biomasse (voir notamment brevet Degrémont FR n°2714667/9315928 déposé le 30.12.93) permettant la concentration de la biomasse active dans l'acidogénèse, ou directement vers l'étape de méthanisation.

US2004/182779 décrit une méthode de traitement de matière organique comprenant une étape de traitement d'acidogénèse dans le réacteur 20 et une étape de méthanisation dans le réacteur 30 (figure 1 de US2004/182779). Un élément de séparation d'acide (élément 22) permet de séparer les matières en suspension avec de l'eau et l'acide volatile. Cependant, ce document ne décrit pas que la séparation est mise en oeuvre par évapo-concentration.

US 5 670 047 décrit uniquement un mode de séparation par flottation de gaz anoxique.

Dans tous les cas la composition des effluents alimentant l'étape de méthanisation hors recyclage éventuel se caractérise par :
→ Les métabolites produits, issus de l'hydrolyse/acidogénèse,
→ Le reste des composés des effluents bruts non transformés ou transformés partiellement comme par exemple les acides gras volatils, alcools, etc... présents initialement, par exemple les composés azotés, phosphorés et soufrés, les anions et cations, et des éléments comme par exemple : huiles, hydrocarbures, tanins, lignines, solvants, sels métalliques, etc...

Certains de ces éléments en fonction de leur concentration comme par exemple les sulfates ou formes soufrées qui conduisent à l'H₂S et HS⁻, par exemple le potassium, le sodium, l'azote ammoniacal présent initialement dans les effluents bruts ou résultant de l'ammonification de l'azote organique, par exemple les tanins, les solvants, etc... peuvent être toxiques pour la méthanisation et inhiber les processus biologiques.

Cette inhibition se traduit par exemple par une forte limitation des rendements d'élimination de la DCO et DBO₅, une réduction importante de la production de biogaz valorisable, la génération de composés H₂S toxiques pour les êtres humains et corrosifs pour les équipements, et des volumes importants de traitement à mettre en oeuvre du fait des cinétiques limitées par les toxiques.

La toxicité de l'effluent à traiter du fait de ces différents composés peut également conduire à l'inadaptation de la méthanisation pour le traitement et donc empêcher l'utilisation d'une technologie performante et, économiquement et du point de vue de l'environnement, très rentable pour les industriels.

Les conséquences sont également très importantes en terme de surcoûts d'investissement pour la mise en oeuvre de la méthanisation mais aussi du fait des traitements complémentaires de la DCO, DBO₅, azote, phosphore, etc... résiduels et des traitements pour l'élimination de H₂S.

D'autre part le manque à gagner lié à une production de biogaz moindre, une valorisation limitée et la consommation énergétique nécessaire pour les traitements complémentaires par voies aérobies, impactent très fortement les coûts de fonctionnement et d'exploitation des installations de traitement des effluents.

L'invention a pour but, surtout, de fournir un procédé de traitement biologique de méthanisation d'effluents industriels ou urbains qui ne présente plus, ou à un degré moindre, les inconvénients exposés précédemment. L'invention vise notamment à fournir une méthanisation à rendement élevé, avec suppression ou forte limitation de produits toxiques en particulier H₂S malgré la présence de composés soufrés, en particulier de sulfates, dans les effluents.

### 3. DESCRIPTION DE L'INVENTION

Selon l'invention, le procédé de méthanisation, à partir d'effluents industriels ou urbains, liquides ou solides, en particulier vinasses issues de la production d'alcools ou de levures, contenant des éléments ou des composés inhibant la méthanisation, comporte :
- l'étape d'hydrolyse et d'acidogénèse, ou d'acidogénèse seule, des effluents avant méthanisation, ladite étape étant contrôlée quant au potentiel d'oxydo-réduction, pour que les réactions biologiques se déroulent en milieu réduit ou légèrement oxydant, et pour produire des métabolites, en particulier acides gras, alcools, mélangés à d'autres composés,
- les métabolites sont soumis à une étape de séparation des autres composés,
- et les métabolites ainsi séparés sont soumis au traitement de méthanisation,
l'étape de séparation des métabolites mélangés consistant en une évapo-concentration, dont les condensats sont soumis à la méthanisation.

De préférence le potentiel d'oxydo-réduction lors de l'étape d'hydrolyse/acidogénèse est maintenu entre - 100mV et 0 mV (potentiel exprimé par rapport à l'électrode de référence à l'hydrogène).

Les concentrats sont utilisés pour diverses applications, notamment amendement agricole.

Il est connu dans l'art que l'étape de séparation des métabolites mélangés peut être effectuée par une nanofiltration dont le filtrat est soumis à la méthanisation.

Le potentiel d'oxydo-réduction dans le réacteur biologique pour le traitement d'hydrolyse/acidogénèse peut être contrôlé et régulé par injection d'air et/ou d'oxygène et/ou d'eau saturée en oxygène, de façon continue ou discontinue.

Le potentiel d'oxydo-réduction dans le réacteur biologique peut aussi être contrôlé et régulé par injection de réactifs choisis parmi :
- le chlore et/ou l'hypochlorite et/ou le brome et/ou l'eau oxygénée,
- les nitrates et/ou les nitrites
- et/ou des désinfectants comprenant les ammoniums quaternaires.

Les métabolites séparés sont traités par méthanisation par exemple en réacteur à lits fluidisés ou expansés de boues fixées ou granuleuses, ou en réacteur UASB (réacteur anaérobie à lit de boues à flux ascendant), ou en réacteur à lit fixé ou en réacteur infiniment mélangé du type contact anaérobie

Le traitement de méthanisation peut être mis en oeuvre dans des réacteurs agités à cultures libres par exemple par le procédé Analift , décrit notamment dans le Mémento Technique de l'Eau de Degrémont, 9^{ème} édition, pages 748-749, ou cultures fixées mobiles par exemple par le procédé Anaflux, décrit notamment dans le Mémento Technique de l'Eau de Degrémont, 9^{ème} édition, pages 755-756 ou immobiles par exemple par le procédé Anafiz, décrit notamment dans le Mémento Technique de l'Eau de Degrémont, 9^{ème} édition, pages 753-754, ou dans un réacteur à lit de boues granuleuses, par exemple par le procédé Anapulse, décrit notamment dans le Mémento Technique de l'Eau de Degrémont, 9^{ème} édition, pages 750-752.

Le procédé peut être mis en oeuvre dans un ou plusieurs étages de traitement ; le pH, le potentiel d'oxydo-réduction, la température, la concentration en biomasse pouvant être différents d'un étage à l'autre.

L'étape d'hydrolyse et/ou d'acidogénèse peut être suivie d'une étape de séparation de l'eau traitée et des boues par décantation et/ou flottation et/ou centrifugation et/ou filtration par membrane de micro, de nano- ou d'ultrafiltration et de recyclage de la biomasse vers le réacteur d'hydrolyse et/ou d'acidogénèse afin de maintenir des concentrations élevées en biomasse entre 2 et 200 g/L.

L'étape d'hydrolyse et/ou d'acidogénèse peut être mise en oeuvre dans des réacteurs permettant un temps de séjour hydraulique des effluents de 6 h à 10 jours, avantageusement de 1 à 3 jours.

L'étape d'hydrolyse et/ou d'acidogénèse peut être mise en oeuvre dans des conditions mésophiles et/ou thermophiles et/ou dans une plage de température comprise entre 15°C et 70°C.

L'étape d'hydrolyse et/ou d'acidogénèse peut être assistée par ajout d'enzymes spécifiques, notamment les lipases, protéases, uréases, les transférases, les hydrolases, etc., et/ou par ajout de cocktails de bactéries et/ou de levures et/ou de champignons.

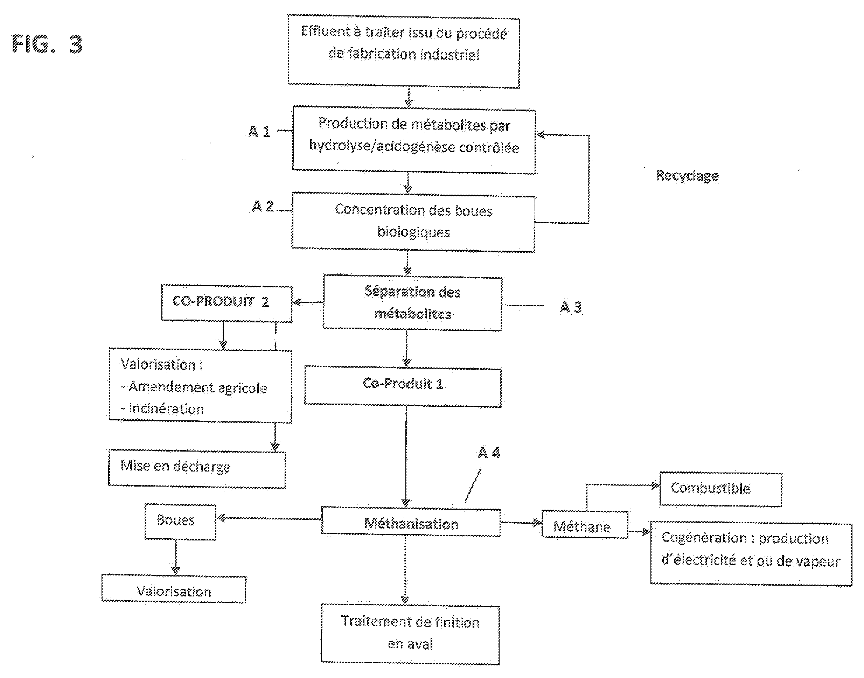

Les métabolites produits et séparés peuvent être récupérés dans le co-produit 1 (condensats) (voir Fig.3 des dessins annexés) et/ou piégés dans des solutions de piégeage adaptées comme par exemple une solution alcaline pour les acides.

Selon l'invention, le procédé est appliqué directement aux effluents, notamment aux vinasses, sans traitement préalable de coagulation.

L'invention décrite plus en détail ci-après permet la séparation des métabolites spécifiques de la méthanisation du reste des composés présents dans les effluents afin de s'affranchir de tous les problèmes de toxicité vis-à-vis de la méthanisation. La dégradation des métabolites par méthanisation est alors optimale (avec des rendements d'élimination de la DCO et DBO₅ jusque 98 %) dans des ouvrages de faible volume et avec une production de biogaz valorisable maximale. La faible quantité de boues produites par la méthanisation et les caractéristiques des effluents traités en terme de : DCO - DBO₅ - H₂S - N - P etc... n'impliquent pas nécessairement de traitements de finition complémentaires et, dans le cas où ils s'avéreraient malgré tout indispensables , ils seraient très limités.

Le procédé de l'invention consiste ainsi en un procédé de traitement biologique des effluents industriels ou urbains, liquides ou solides par exemple ceux :
- de i'agro-aiimentaire : lévureries, brasseries, fromageries, salaisons, abattoirs, conserveries, production d'arômes alimentaires, charcuteries, distilleries, laiteries, production d'aliments pour animaux, production de glaces, caves vinicoles, production d'alcools, d'acide citrique, d'acide ascorbique, gélatine, etc.,
- des biotechnologies : production de milieux de culture, de vitamines, d'antibiotiques, d'acides aminés, d'anti-inflammatoires comme la cortisone, etc.,
- des biocarburants : production de bioéthanol et d'alcools, production de diesters, etc.,
- des déchetteries comme par exemple : les déchets verts, les graisses et les boues de station d'épuration d'eaux urbaines et industrielles,
- de la papeterie : pâte à papier, papier recyclé, etc.,
- de la chimie, de la pétrochimie ; de la pharmacie,
- des matières de vidange comme par exemple : fosses septiques, lisiers de porc, etc...,
- etc...
ces effluents industriels ou urbains contenant par exemple ;
- des anions par exemple : sulfates, nitrates, nitrites, chlorures, bromures, phosphates, etc.,
- et/ou des cations par exemple : potassium, sodium, calcium, magnésium, ammonium, etc.,
- et/ou de l'azote organique sous forme NTK,
- et/ou des graisses et/ou matières en suspension
- et/ou des huiles et/ou des hydrocarbures,
- et/ou des acides bromiques,
- et/ou des tanins,
- et/ou des lignines
- et/ou des solvants
- et/ou des sulfites
- et/ou des thiosulfates, etc.,
- et/ou des sels métalliques,
- et/ou des hydrates de carbone (amidon, fécule, gluten, etc.),
- et/ou des lipides
- et/ou des sucres
- et/ou des protéines
- et/ou des acides aminés
- et/ou de la cellulose
- etc...

Le procédé de l'invention consiste également en un procédé de traitement biologique des effluents concentrés en COT, DBO₅, DCO et dont la DCO est comprise entre 5 et 3600 g/L (grammes par litre).

Le procédé de l'invention consiste également en un procédé de traitement biologique conduisant à la production de métabolites de la méthanisation comme les alcools (méthanol, éthanol, etc.), comme les acides gras volatils comme l'acide acétique, butyrique, isobutyrique, propionique, valérique, isovalérique ; l'acide lactique ; l'acide formique, etc., des aldéhydes et cétones.

Le procédé de l'invention consiste également en un procédé de traitement biologique couplé à une ou plusieurs étapes de séparation permettant de séparer les métabolites, produits par voies biologiques à partir des composés biodégradables contenus dans l'effluent, du substrat résiduel non hydrolysable, des effluents industriels ou urbains.

La séparation est réalisée à partir de la liqueur provenant du traitement biologique, et ayant subi ou non une étape de concentration des boues biologiques, par exemple par décantation, flottation, centrifugation, filtration par membrane de micro, d'ultra ou nanofiltration permettant le recyclage des boues biologiques vers l'hydrolyse/acidogénèse. Cette séparation est réalisée avantageusement par évapo-concentration, et/ou stripping (c'est-à-dire entraînement) à chaud ou à froid par un gaz ou un mélange de gaz comme par exemple le CO₂, le CH₄, l'azote, l'air, etc., et/ou par entraînement à la vapeur, et/ou par distillation et éventuellement par nanofiltration.

Le procédé de traitement biologique, selon l'invention, met en oeuvre une fermentation du type hydrolyse + acidogénèse ou acidogénèse seule ; les réactions biologiques peuvent se dérouler en milieu réduit ou légèrement oxydant entre -350 mV et +300 mV (potentiel exprimé par rapport à l'électrode de référence à l'hydrogène).

Le procédé de traitement biologique peut être suivi d'une étape de séparation par décantation et/ou flottation et/ou centrifugation et/ou filtration par membrane de micro, de nano- ou d'ultrafiltration et de recyclage de la biomasse vers le réacteur d'hydrolyse et/ou d'acidogénèse afin de maintenir des concentrations élevées en biomasse entre 2 et 200 g/L.

### 4. BREVE DESCRIPTION DES FIGURES DES DESSINS

D'autres caractéristiques et avantages de l'invention apparaîtront à la lecture de la description qui va suivre, donnée à titre d'exemples non limitatifs, pour la compréhension de laquelle on se reportera aux dessins annexés dans lesquels :
Fig.1 est un schéma des étapes d'un procédé classique de méthanisation de matières organiques complexes ;
Fig.2 est un schéma illustrant les voies métaboliques principales des bactéries produisant des acides.
Fig.3 est un tableau illustrant le procédé selon l'invention, et
Fig. 4 est un diagramme illustrant l'évolution de la concentration en AGV, en grammes / litre, portée en ordonnée, en fonction du temps, en jours, porté en abscisse.

Fig.3 est une représentation schématisée des grands groupes principaux d'opérations que comporte généralement un exemple de procédé selon l'invention, avec production d'un co-produit 2 (concentrat) et d'un co-produit 1 (condensats) apte à être méthanisé, la séparation des co-produits intervenant avant méthanisation.

L'effluent à traiter issu d'un procédé de fabrication industriel, ou un effluent urbain, est introduit lors d'une étape A1 dans un bassin couvert d'acidification (hydrolyse), généralement brassé, travaillant en anoxie pour une production de métabolites par hydrolyse / acidogénèse contrôlée. L'acidification, par traitement biologique, est normalement réalisée avec des cultures libres, mais il est possible de mettre en oeuvre des cultures fixées. Une étape suivante A2 de concentration des boues biologiques est généralement prévue avec recyclage d'une fraction des boues vers l'étape A1.

L'hydrolyse /acidogénèse, selon l'invention, est contrôlée pour limiter la baisse du potentiel redox et éviter le démarrage d'une sulfato-réduction qui produirait du SH₂. Le potentiel redox est maintenu de préférence entre -100mV et 0mV. Le pH peut éventuellement remonter de 5 à 6 lors de l'hydrolyse.

Le diagramme de Fig.4 de la concentration de l'effluent en AGV après hydrolyse/acidogénèse, reste pratiquement constant après 3 jours, la concentration en AGV exprimée en g CH₃COOH/L ayant atteint 40g/L.

Le potentiel d'oxydo-réduction dans le bassin formant réacteur biologique pour le traitement d'hydrolyse/acidogénèse peut être contrôlé et régulé par injection d'air et/ou d'oxygène et/ou d'eau saturée en oxygène, de façon continue ou discontinue.

Le potentiel d'oxydo-réduction dans le réacteur biologique peut aussi être contrôlé et régulé par injection de réactifs choisis parmi :
- le chlore et/ou l'hypochlorite et/ou le brome et/ou l'eau oxygénée,
- les nitrates et/ou les nitrites,
- et/ou des désinfectants comprenant les ammoniums quaternaires.

La liqueur et la fraction des boues non recyclée est soumise, selon l'invention, à une étape A3 de séparation des métabolites donnant un co-produit 1 et un co-produit 2.

L'étape de séparation A3 consiste avantageusement en une étape d'évapo-concentration donnant comme co-produit 1 un condensat contenant des métabolites et de l'eau, et comme co-produit 2 un concentrat avec les composés gênants pour la méthanisation.

La séparation pourrait être effectuée par nanofiltration, auquel cas une évapo-concentration ne serait plus nécessaire.

La séparation du co-produit 2 (concentrat) et du co-produit 1 (condensats) est avantageusement effectuée dans des conditions telles que la teneur massique en matières en suspension (MES) dans la phase liquide (co-produit 1) avant méthanisation, est inférieure à 0,1 % et plus particulièrement 0,01 %. Cette étape permet de concentrer les métabolites spécifiques de la méthanisation d'une part et de s'affranchir d'autre part des MES et des composés indésirables.

Le co-produit 1 est soumis lors d'une étape A4 à un traitement de méthanisation dans un réacteur fermé :
- soit à travers un lit de boues granuleuses, fluidisées, recirculées ou expansées en un ou plusieurs étages ou de boues floculées, selon un flux ascendant de la phase liquide du co-produit 1
- soit au moyen d'un réacteur à lit fluidisé de biomasse (cultures fixées sur un support libre mobile),
- soit au moyen d'un réacteur à lit fixé de biomasse (cultures fixées sur un support immobile),
- soit au moyen d'un réacteur à UASB ou infiniment mélangé de type contact anaérobie (cultures libres).

Un exemple de réacteur pour traitement de méthanisation à travers un lit de boues granuleuses est connu sous le nom de « Anapulse », et est décrit notamment dans le Mémento Technique de l'Eau de Degrémont, 9^{ème} édition, pages 750-752.

Un exemple de réacteur à cultures fixées sur un support libre mobile est connu sous le nom de « Anaflux » et est décrit notamment dans le Mémento Technique de l'Eau de Degrémont, 9^{ème} édition, pages 755-756.

La méthanisation peut aussi être réalisée dans un réacteur de type « Anafiz » qui est un réacteur à culture fixée sur un garnissage (par exemple en plastique, polypropylène, polyéthylène), ordonné ou en vrac. Un réacteur de ce type est décrit notamment dans le Mémento Technique de l'Eau de Degrémont 9^{ème} édition, pages 753 -754.

Le méthane produit est utilisé comme combustible et/ou en cogénération pour production d'électricité et/ou vapeur. Les boues provenant de la méthanisation sont destinées à la valorisation : amendement agricole, ou à l'incinération. Un traitement de finition peut être prévu en aval de la méthanisation pour le rejet liquide.

Le co-produit 2 est destiné à la valorisation : amendement agricole, incinération, ou à la mise en décharge.

### 5. NORMES ANALYTIQUES AFNOR

AGV : Acides Gras Volatils (analyse chromatographique)
Ca : Calcium (NF EN ISO 11885)
DBO₅ : Demande Biochimique en Oxygène pendant cinq jours (NF EN 1899-1, NF EN 1899-2)
DCO: Demande Chimique en Oxygène (NFT 90-101, ISO 6060: 1989)
K⁺ : Potassium (NF EN ISO 11885, NF EN ISO 14911)
MES : Matières En Suspension, Matières En Suspension Totales (NF EN 872, NFT 90-105-2)
Mg²⁺ : Magnésium (NF EN ISO 11885)
MS : Matières Sèches (NF U 44-171)
MVS : Matières Volatiles en Suspension (NF U 44-171)
Na⁺ : Sodium (NF EN ISO 11885, NFT 90-019, NF EN ISO 14911)
NGL : Azote global (NF EN ISO 11905-1)
NH₄⁺ : Ammonium (NF T 90-015-1 NF T90-015-2)
NO₂⁻ : Nitrite (NF EN ISO 13 395, NF EN 26777)
NO₃⁻ : Nitrate (NF EN ISO 13 395, NF EN ISO 10304-1)
P ortho : Orthophosphate (NF EN 1189, NF EN ISO 6878)
P total : Phosphore total (NF EN 1189, NF EN ISO 6878)
pH : potentiel d'Hydrogène (NFT 90-008)
SO₄²⁻ : Sulfate (Norme NF EN ISO 10304-1)
TH : Titre Hydrotimétrique (NFT 90-003)
TA : Titre Alcalimétrique (NF EN ISO 9963-)
TAC : Titre Alcalimétrique Complet (NF EN ISO 9963-)

### 6. RESULTATS OBTENUS

### 6.1 Spectre de composition acceptable pour l'effluent brut en amont du procédé

Le tableau 1 décrit la composition de l'effluent brut à traiter.

**Tableau 1 :**

| Paramètres | EFFLUENT BRUT |
|---|---|
| PH | 1 à 14 |
| TH (°F) | 0 à 2500 |
| TA (°F) | 0 à 2500 |
| TAC (°F) | 0 à 2500 |
| Ca (g/l) | 0 à 10 |
| Mg²⁺ (g/l | 0 à 10 |
| DCO (g/l) | 5 à 3600 |
| DBO₅ (g/l) | 2 à 2800 |
| AGV (g/l) | 0 à 15 |
| MES (g/l) | 0,1 à 150 |
| NH₄⁺ (g/l) | 0 à 25 |
| NGL (g/l) | 0 à 50 |
| Ptotal (g/l) | 0 à 15 |
| Portho (g/l) | 0 à 15 |
| NO₂ (mg/l) | 0 à 5000 |
| NO₃ (mg/l) | 0 à 10000 |
| SO₄²⁻ (g/l) | 0,5 à 200 |
| K⁺ (g/l) | 0,5 à 30 |
| Chlorure (g/l) | 0,1 à 100 |

### 6.2 Caractéristiques de l'effluent industriel testé suivant le procédé à titre d'exemple

**Tableau 2 : Caractéristiques de l'effluent industriel testé suivant le procédé à titre d'exemple.**

| Paramètres | EFFLUENT INDUSTRIEL TESTE |
|---|---|
| PH | 3 à 5 |
| TH (°F) | 600 |
| TA (°F) | 1000 à 1600 |
| TAC (°F) | 1000 à 1600 |
| Ca (g/l) | 8 à 12 |
| Mg²⁺ (g/l | 8 à 12 |
| DCO (g/l) | 50 à 60 |
| DBO₅ (g/l) | 33 à 42 |
| AGV (g/l) | 4 à 5 |
| MES (g/l) | 1,6 à 2 |
| NH₄⁺ (g/l) | 100 à 300 |
| NGL (g/l) | 3 à 5 |
| P total (g/l) | 200 à 400 |
| P ortho (g/l) | 160 à 250 |
| NO₂ (mg/l) | 160 à 250 |
| NO₃ (mg/l) | 160 à 250 |
| SO₄²⁻ (g/l) | 12 à 16 |
| K⁺ (g/l) | 16 à 24 |
| Chlorure (g/l) | 4 à 10 |

Les essais ont été réalisés à différentes échelles :
- laboratoire
- pilote
- semi-industriel

### 6.3 Résultats obtenus après traitement par hydrolyse/acidogénèse contrôlée 6.3.1 Production d'AGV par hydrolyse/acidogénèse contrôlée

Les concentrations de l'effluent en acides gras volatils après hydrolyse/acidogénèse contrôlée sont présentées dans le tableau qui suit. La concentration en AGV exprimée en g CH₃COOH/L atteint 40 g/L après 3 jours.

### 6.3.2 Composition des acides gras volatils produits

Les pourcentages d'acides gras volatils produits sont présentés comme exemple dans le tableau 3. La colonne de droite donne le pourcentage en masse d'acide de la colonne de gauche dans les AGV :

**Tableau 3 :**

| | Formule chimique | Pourcentage des AGV produits |
|---|---|---|
| Acide acétique | CH₃COOH | 20 à 40 % |
| Acide propionique | CH₃CH₂COOH | 15 à 30 % |
| Acide butyrique et isobutyrique | CH₃CH₂CH₂COOH | 15 à 35 % |
| Acide valérique | CH₃CH₂CH₂CH₂COOH | 0,1 à 5 % |
| Acide iso-valérique | CH₃CH₃CHCH₂COOH | 2 à 10 % |

### 6.3.3 Concentrations en DCO liée aux AGV produits

Les concentrations en DCO liées aux AGV produits après traitement par hydrolyse/acidogénèse contrôlée sont présentées comme exemple dans le tableau 4. D'autres types de métabolites sont également générés : par exemple des alcools, des esters, des éthers, autres. La colonne de droite donne le pourcentage de DCO apportée par l'acide mentionné dans la colonne de gauche.

**Tableau 4 :**

| Paramètres | Formule chimique | Pourcentage de DCO |
|---|---|---|
| Acide acétique | CH₃COOH | 30 à 50 % |
| Acide propionique | CH₃CH₂COOH | 25 à 35 % |
| Acide butyrique et isobutyrique | CH₃CH₂CH₂COOH | 5 à 15 % |
| Divers (alcools, autres) | | 5 % |
| Acide iso-valérique | CH₃CH₃CHCH₂COOH | 2 à 10 % |

La concentration en DCO biodégradable liée aux AGV produits au cours de l'étape d'hydrolyse/acidogénèse contrôlée est de l'ordre de 80 % de la DCO initialement présente dans l'effluent brut.

### 6.3.4 Séparation des métabolites produits lors de l'étape d'hydrolyse/acidogénèse contrôlée

Le tableau 5 présente les conditions opératoires pour la mise en oeuvre de l'étape de séparation des métabolites produits. Les métabolites produits et séparés sont récupérés dans le co-produit 1 (condensats) et/ou piégés dans des solutions de piégeage adaptées comme par exemple une solution alcaline pour les acides.

**Tableau 5 :**

| Paramètres | Séparation des métabolites |
|---|---|
| pH | 3-9 |
| t (°C) | Point d'ébullition de l'effluent selon conditions opératoires |
| Facteur de concentration | 30 à 80 % |

L'étape de séparation des métabolites pouvant s'effectuer par exemple par évapo-concentration, aboutit d'abord à la production de condensats (co-produit 1) et de concentrat (co-produit 2). Ces condensats (co-produit 1) sont ensuite traités par méthanisation.

**Tableau 6 : Composition des condensats issus de l'étape de séparation**

| Paramètres | Condensats obtenus après séparation |
|---|---|
| pH | 2 à 9 |
| DCO (g/l) | 50 |
| DBO₅ (g/l) | 33 |
| TH (°F) | < 5°F |
| TA (°F) | < 5°F |
| TAC (°F) | < 5°F |
| NGL x | < 150 mg/l |
| NH₄ x | < 120 mg/l |
| P ortho x | < 20 mg/l |
| P total x | < 20 mg/l |
| MES x | < 50 mg/l |
| Sulfates x | < 10 mg/l |
| Potassium x | < 10 mg/l |
| Calcium | < 20 mg/l |
| Sodium | < 20 mg/l |
| Magnésium | < 20 mg/l |
| Salinité | < 1 g/l |
| Nitrate | < 20 mg/l |
| Nitrite | < 5 mg/l |

Outre les condensats (co-produit 1), l'évapo-concentration produit des résidus (co-produit 2). Ces résidus pourront être valorisés (par exemple pour l'amendement des sols agricoles, réutilisation pour d'autres procédés éventuellement pour alimentation bétail,ou détruits par incinération.

Après production de métabolites et séparation par exemple par évapo-concentration, le co-produit 1 (condensats) contient des matières très facilement biodégradables par exemple des acides gras volatils , alcools, des esters, des éthers, ou autres, qui permettent de mettre en oeuvre des réacteurs de méthanisation fortes charges (jusqu'à 60 kg DCO/m³/j), par exemple de type Anaflux, à des rendements élevés : jusqu'à 98 %.

La séparation des métabolites produit par l'hydrolyse/acidogénèse permet un traitement par méthanisation à très haut rendement ainsi qu'une réduction importante du temps de séjour et de la surface au sol occupée.

La méthanisation de l'effluent tel quel selon les procédés conventionnels nécessiterait une dilution importante de l'effluent brut avec de l'eau non contaminée et entraînerait la mise en oeuvre d'ouvrages de volumes nettement plus importants (quatre à dix fois en fonction de la dilution) afin de réduire les seuils de toxicité des composés. Ce traitement par méthanisation à très haut rendement du co-produit 1 (condensats) permet ainsi réduire la surface au sol de façon très importante.

L'étape de séparation des métabolites pouvant s'effectuer par exemple par évapo-concentration permet de séparer le co-produit 1 (condensats facilement biodégradables), du co-produit 2 (concentrat) riches en sulfates et autres composés toxiques pour les bactéries comme par exemple le potassium, le sodium, le calcium, le magnésium, la salinité, le NH₄⁺, etc.

Ceci conduit à la maîtrise des formes H₂S habituellement produites durant l'étape de méthanisation à partir des sulfates contenus dans l'effluent à traiter et permet de réduire fortement les coûts d'investissement liés aux équipements supplémentaires à mettre dans la solution classique comme par exemple le lavage du biogaz, l'utilisation de matériaux adaptés à la corrosion, traitement des odeurs.

Un avantage important du procédé concerne l'optimisation des conditions Hygiène Sécurité Environnement et la réduction du temps d'exploitation de l'installation et les dépenses associées.

L'invention permet des conditions très optimisées pour la méthanisation, en particulier :
- une teneur massique en matières en suspension dans le co-produit 1 (condensats) avant méthanisation inférieure à 0,1 % et plus particulièrement 0,01 % (Analyse suivant la norme NF EN 872),
- une teneur en sulfates, potassium, calcium, sodium, magnésium et autres sels dans le co-produit 1 (condensats) inférieure à 0,01 % et plus particulièrement inférieure à 0,005 %.

La phase liquide provenant de la séparation est soumise à un traitement par méthanisation,
- soit à travers un lit de boues granuleuses, fluidisées, recirculées ou expansées en un ou plusieurs étages ou de boues floculées, selon un flux ascendant de la phase liquide, en particulier celle du co-produit 1,
- soit au moyen d'un réacteur à lit fluidisé de biomasse (cultures fixées sur un support libre mobile),
- soit au moyen d'un réacteur à lit fixé de biomasse (cultures fixées sur un support immobile),
- soit au moyen d'un réacteur à UASB ou infiniment mélangé de type contact anaérobie (cultures libres).

Un exemple de réacteur pour traitement de méthanisation à travers un lit de boues granuleuses est connu sous le nom de « Anapulse », et est décrit notamment dans le Mémento Technique de l'Eau de Degrémont, 9^{ème} édition, pages 750-752.

Un exemple de réacteur à cultures fixées sur un support libre mobile est connu sous le nom de « Anaflux » et est décrit notamment dans le Mémento Technique de l'Eau de Degrémont, 9^{ème} édition, pages 755-756.

La méthanisation peut aussi être réalisée dans un réacteur de type « Anafiz » qui est un réacteur à culture fixée sur un garnissage (par exemple en plastique, polypropylène, polyéthylène), ordonné ou en vrac. Un réacteur de ce type est décrit notamment dans le Mémento Technique de l'Eau de Degrémont 9^{ème} édition, pages 753 -754.

En conclusion, l'invention concerne la production et la séparation de métabolites spécifiques de la méthanisation. La production des métabolites est réalisée dans un réacteur d'hydrolyse/acidogénèse mésophile ou thermophile optimisé avec régulation du potentiel d'oxydo-réduction.

La séparation des métabolites par évapo-concentration permet de récupérer les métabolites dans le co-produit 1 (condensats) et/ou solution de piégeage.

Le co-produit 1 (condensats) et/ou solutions de piégeage sont ensuite traités par méthanisation pour produire du biogaz valorisable.

Selon le procédé de l'invention, les métabolites de la méthanisation sont séparés de tout le reste. La phase de séparation des métabolites est primordiale. L'étape d'acidogénèse séparée de l'étape de méthanogénèse et contrôlée par le potentiel d'oxydo-réduction en amont de la méthanisation pour produire des métabolites spécifiques est obligatoire avant l'étape de séparation et de méthanisation.

L'invention conduit à une optimisation des rendements d'élimination de la DCO, DBO₅ et de la production de biogaz, à une réduction de la production de boues, des volumes de traitement à mettre en oeuvre, et des coûts d'investissement. Elle permet aussi de s'affranchir des contraintes liées aux nuisances olfactives, de toxicité pour l'homme et de corrosion des équipements.

La méthanisation fournit du biogaz, principalement du méthane, qui peut être utilisé en particulier pour produire de l'énergie électrique, et/ou de la vapeur.

## Revendications

1. Procédé de méthanisation, à partir d'effluents industriels ou urbains, liquides ou solides, en particulier vinasses issues de la production d'alcools ou de levures, contenant des éléments ou des composés inhibant la méthanisation, comportant :
- l'étape d'hydrolyse et d'acidogénèse, ou d'acidogénèse seule, des effluents avant méthanisation, ladite étape étant contrôlée quant au potentiel d'oxydoréduction, pour que les réactions biologiques se déroulent en milieu réduit ou légèrement oxydant, et pour produire des métabolites, en particulier acides gras, alcools, mélangés à d'autres composés,
- les métabolites sont soumis à une étape de séparation des autres composés,
- et les métabolites ainsi séparés sont soumis au traitement de méthanisation,
**caractérisé en ce que** l'étape de séparation des métabolites mélangés consiste en une évapo-concentration, dont les condensats sont soumis à la méthanisation.

2. Procédé selon la revendication 1, **caractérisé en ce que** le potentiel d'oxydo-réduction lors de l'étape hydrolyse et acidogénèse ou acidogénèse est maintenu entre - 100mV et 0 mV (potentiel exprimé par rapport à l'électrode de référence à l'hydrogène).

3. Procédé selon l'une quelconque des revendications précédentes, **caractérisé en ce que** le potentiel d'oxydo-réduction dans le réacteur biologique est contrôlé et régulé par injection d'air et/ou d'oxygène et/ou d'eau saturée en oxygène de façon continue ou discontinue,

4. Procédé selon l'une quelconque des revendications précédentes, **caractérisé en ce que** le potentiel d'oxydo-réduction dans le réacteur biologique est contrôlé et régulé par injection de réactifs choisis parmi :
- le chlore et/ou l'hypochlorite et/ou le brome et/ou l'eau oxygénée,
- les nitrates et/ou les nitrites,
- et/ou des désinfectants comprenant les ammoniums quaternaires.

5. Procédé selon l'une quelconque des revendications précédentes, **caractérisé en ce que** le traitement d'hydrolyse/acidogénèse ou acidogénèse seule est mis en oeuvre dans un réacteur agité à cultures libres, ou à cultures fixées mobiles, ou immobiles, ou dans un réacteur à lit de boues granuleuses.

6. Procédé selon l'une quelconque des revendications précédentes, **caractérisé en ce qu'**il est mis en oeuvre dans plusieurs étages de traitement, le pH, le potentiel d'oxydo-réduction, la température, la concentration en biomasse pouvant être différents d'un étage à l'autre.

7. Procédé selon l'une quelconque des revendications précédentes, **caractérisé en ce que** l'étape hydrolyse et acidogénèse ou acidogénèse seule est suivie d'une étape de séparation par décantation et/ou flottation et/ou centrifugation et/ou filtration par membrane de micro, de nano- ou d'ultrafiltration et de recyclage de la biomasse vers le réacteur afin de maintenir des concentrations élevées en biomasse entre 2 et 200 g/L.

8. Procédé selon l'une quelconque des revendications précédentes, **caractérisé en ce qu'**il est mis en oeuvre dans des réacteurs permettant un temps de séjour hydraulique des effluents de 6 h à 10 jours.

9. Procédé selon l'une quelconque des revendications précédentes, **caractérisé en ce que** l'étape d'hydrolyse et acidogénèse ou acidogénèse seule est mise en oeuvre dans des conditions mésophiles et/ou thermophiles et/ou dans une plage de température comprise entre 15°C et 70°C.

10. Procédé selon l'une quelconque des revendications précédentes, **caractérisé en ce que** l'étape d'hydrolyse et acidogénèse ou acidogénèse seule est assistée par ajout d'enzymes spécifiques comme par exemple les lipases, protéases, uréases, les transférases et les hydrolyses et/ou par ajout de cocktails de bactéries et/ou de levures et/ou de champignons.

## Patentansprüche

1. Verfahren zur Methanisierung ausgehend von industriellen oder kommunalen Abwässern oder Abfällen, insbesondere Schlempen aus der Produktion von Alkoholen oder Hefen, die Elemente oder Verbindungen enthalten, die die Methanisierung hemmen, umfassend:
- den Schritt der Hydrolyse und Acidogenese oder nur der Acidogenese der Abwässer oder Abfälle vor der Methanisierung, wobei dieser Schritt bezüglich des Redoxpotentials gesteuert wird, damit die biologischen Reaktionen im reduzierenden oder leicht oxidierenden Medium ablaufen, und zur Herstellung von Metaboliten, insbesondere Fettsäuren, Alkohole, die mit anderen Verbindungen gemischt sind;
- die Metabolite werden einem Schritt der Abtrennung von den anderen Verbindungen unterzogen;
- und die so abgetrennten Metabolite werden einer Methanisierungsbehandlung unterzogen;
**dadurch gekennzeichnet, dass** der Schritt der Abtrennung der gemischten Metabolite aus einer Eindampfung besteht, deren Kondensate der Methanisierung unterzogen werden.

2. Verfahren gemäß Anspruch 1, **dadurch gekennzeichnet, dass** das Redoxpotential im Schritt der Hydrolyse und Acidogenese oder der Acidogenese zwischen -100 mV und 0 mV gehalten wird (Potential ausgedrückt in Bezug auf die Wasserstoffreferenzelektrode).

3. Verfahren gemäß einem der vorstehenden Ansprüche, **dadurch gekennzeichnet, dass** das Redoxpotential im biologischen Reaktor durch kontinuierliches oder diskontinuierliches Einleiten von Luft und/oder Sauerstoff und/oder sauerstoffgesättigtem Wasser gesteuert und reguliert wird.

4. Verfahren gemäß einem der vorstehenden Ansprüche, **dadurch gekennzeichnet, dass** das Redoxpotential im biologischen Reaktor durch Einleiten von Reaktanten gesteuert und reguliert wird, die ausgewählt sind aus:
- Chlor und/oder Hypochlorit und/oder Brom und/oder Wasserstoffperoxid;
- Nitrate und/oder Nitrite;
- und/oder Desinfektionsmittel, die quartäre Ammoniumverbindungen umfassen.

5. Verfahren gemäß einem der vorstehenden Ansprüche, **dadurch gekennzeichnet, dass** die Behandlung durch Hydrolyse/Acidogenese oder nur Acidogenese in einem Rührreaktor mit freien Kulturen oder beweglichen oder unbeweglichen fixierten Kulturen oder in einem Wirbelschichtreaktor durchgeführt wird.

6. Verfahren gemäß einem der vorstehenden Ansprüche, **dadurch gekennzeichnet, dass** es in mehreren Behandlungsschritten durchgeführt wird, wobei der pH-Wert, das Redoxpotential, die Temperatur und die Konzentration an Biomasse von einem Schritt zum anderen unterschiedlich sein können.

7. Verfahren gemäß einem der vorstehenden Ansprüche, **dadurch gekennzeichnet, dass** auf den Schritt der Hydrolyse und Acidogenese oder nur der Acidogenese ein Schritt der Trennung durch Dekantieren und/oder Flotation und/oder Zentrifugation und/oder Filtration mit einer Mikro- oder Nanomembran oder Ultrafiltration und der Rückführung der Biomasse zum Reaktor, um die erhöhten Konzentrationen der Biomasse zwischen 2 und 200 g/l zu halten, folgt.

8. Verfahren gemäß einem der vorstehenden Ansprüche, **dadurch gekennzeichnet, dass** es in Reaktoren durchgeführt wird, die eine hydraulische Verweilzeit der Abwässer von 6 h bis 10 Tagen ermöglichen.

9. Verfahren gemäß einem der vorstehenden Ansprüche, **dadurch gekennzeichnet, dass** der Schritt der Hydrolyse und Acidogenese oder nur der Acidogenese unter mesophilen und/oder thermophilen Bedingungen und/oder in einem Temperaturbereich zwischen 15 °C und 70 °C durchgeführt wird.

10. Verfahren gemäß einem der vorstehenden Ansprüche, **dadurch gekennzeichnet, dass** der Schritt der Hydrolyse und Acidogenese oder nur der Acidogenese durch Zugabe von spezifischen Enzymen, wie zum Beispiel Lipasen, Proteasen, Ureasen, Transferasen und Hydrolasen und/oder durch Zugabe von Cocktails von Bakterien und/oder Hefen und/oder Pilzen unterstützt wird.

## Claims

1. Method of anaerobic digestion, from liquid or solid, industrial or urban effluents, in particular vinasses from alcohol or yeast production, containing the elements or the compounds inhibiting the anaerobic digestion, including:
- a step of acidogenesis and of hydrolysis, or of acidogenesis alone, of effluents before anaerobic digestion, said step being controlled for the oxydo-reduction potential, for that biological reactions are carried out in reduced or slight oxidizing medium, and for produce metabolites, in particular fatty acid, alcohol, mixed with other compounds,
- the metabolites are subjected to a separation step of other compounds,
- and the metabolites thus separated are subjected to a treatment of anaerobic digestion,
**characterised in that** the separation step of mixed metabolites consists of an evapo-concentration, wherein the condensates are subjected to an anaerobic digestion.

2. Method according to claim 1, **characterised in that** the oxydo-reduction potential during the step of acidogenesis and of hydrolysis, or of acidogenesis alone is maintained between -100 mV and 0 mV (potential expressed with respect to reference electrode of hydrogen.

3. Method according to any one of previous claims, **characterised in that** the oxydo-reduction potential in biological reactor is controlled and regulated by injection of air and/or of oxygen and/or of water saturated with oxygen in a continuous or discontinuous way.

4. Method according to any one of previous claims, **characterised in that** the oxydo-reduction potential in biological reactor is controlled and regulated by injection of reagents chosen from:
- chlorine and/or hypochlorite and/or bromine and/or oxygenated water,
- nitrates and/or nitrites,
- and/or the disinfectants comprising quaternary ammoniums.

5. Method according to any one of previous claims, **characterised in that** the treatment of acidogenesis and of hydrolysis, or of acidogenesis alone is carried out in a stirred reactor of free cultures, or of mobile or immobile fixed cultures, or in a granular sludge-bed reactor.

6. Method according to any one of previous claims, **characterised in that** it is carried out in multiple steps of treatment, the pH, the oxydo-reduction potential, the temperature, the biomass concentration being able to be different from a step to another.

7. Method according to any one of previous claims, **characterised in that** the step of acidogenesis and of hydrolysis, or of acidogenesis alone is followed by a step of separation by setting and/or flotation and/or centrifugation and/or filtration by micro, nano- or ultrafiltration membrane and the recycling of the biomass to the reactor in order to maintain the high concentrations of biomass between 2 and 200 g/L.

8. Method according to any one of previous claims, **characterised in that** it is carried out in the reactors allowing a hydraulic retention time of effluents from 6h to 10 days.

9. Method according to any one of previous claims, **characterised in that** the step of acidogenesis and hydrolysis, or acidogenesis alone is carried out in mesophilic and/or thermophilic conditions and/or in a temperature range from 15°C to 70 °C.

10. Method according to any one of previous claims, **characterised in that** the step of acidogenesis and hydrolysis, or acidogenesis alone is assisted by addition of specific enzymes such as example the lipases, proteases, ureases, the transferases and the hydrolyses, and/or by addition of cocktails of bacteria and/or of yeasts and/or of fungus.
